# EUROPEAN PATENT APPLICATION

(11) **EP 1 570 850 A1**
(43) Date of publication of application: **07.09.2005**
(21) Application number: 04030859.5
(22) Date of filing: 28.12.2004
(51) Int. Cl.: A61K 31/7056, A61K 31/327, A61P 17/00, A61K 47/08

(54) **Topical formulations for treatment of rosacea**

(30) Priority: 04.03.2004 US 791862
(71) Applicant: STIEFEL LABORATORIES, INC., Coral Gables, FL 33134 (US)
(72) Inventor: Popp, Karl F., Schodack Landing New York 12156 (US)
(74) Representative: Welch, Andreas

(57) **Abstract**

Topical composition comprising a storage-stable mixture of a benzoyl peroxide dispersion and clindamycin or pharmaceutically acceptable salts or esters thereof for treating rosacea, the reduction or elimination of *Demodex folliculorum* organisms from affected skin areas, thereby reducing clinical signs of rosacea potentially due to allergic and vasomotor responses of the body to the organism in susceptible persons.

## Description

This application claims priority to U.S. Patent Application Serial No. 10/791,862, filed on March 4, 2004, the contents of which are hereby incorporated by reference in their entirety.

### FIELD OF THE INVENTION

The present subject matter relates to methods of using a topical composition comprising a storage-stable mixture of a benzoyl peroxide dispersion and clindamycin or pharmaceutically acceptable salts or esters thereof for treating rosacea. These methods also contemplate the reduction or elimination of *Demodex folliculorum* organisms from affected skin areas, thereby reducing clinical signs of rosacea potentially due to allergic and vasomotor responses of the body to the organism in susceptible persons.

### BACKGROUND OF THE INVENTION

Rosacea, originally termed acne rosacea, is a chronic inflammatory skin condition affecting the eyelids and face, particularly the cheeks, chin, nose, and forehead, of certain middle-aged adults. Common clinical signs include erythema (redness), prominent vascularization, dryness, papules, pustules, swelling, telangiectasia, lesions, inflammation, infection, enlarged nasal area, hypertrophy of the sebaceous glands, and nodules either singly or in combination in the involved skin areas, primarily in the central areas of the face. Some of these clinical signs, in particular the ery-thema, are thought to be caused by the dilation of blood vessels. Rosacea may further be characterized by flushing and blushing. In rare instances, rosacea may also occur on the trunk and extremities, such as the chest, neck, back, or scalp.

Eyelids affected by rosacea may be manifested by mild conjunctival irritation or inflammation of the meibomian (oil) glands on the eyelid margin. Chronic eyelid irritation can result in loss of eyelashes. No visual impairment accompanies the eyelid irritation.

Rosacea, in mild form, brings about a slight flushing of the nose and cheeks and, in some cases, the forehead and chin. However, in a severe form, lesions appear which are deep or purplish red and which include a chronic dilation of the superficial capillaries, i.e. telangiectasia. Also, in severe form, inflammatory acneiform pustules are present. Chronic involvement of the nose with rosacea in men can cause a bulbous enlargement known as rhinophyma. However, women are twice as likely as men to have rosacea. In women, this rhinophyma often takes the form of pimples and redness of or near the nose. Similarly, women are three times more likely than men to exhibit symptoms of perioral dermatitis, where redness and a rash appear above the upper lip attaching into the nose.

Another acute form of rosacea is known as granulomatous rosacea and, as such, is considered to be a distinctive form of the papular aspect of the disease. Therein, discreet pustules appear as yellowish brown nodules and as epithelioid cell granulomatous.

Rosacea may be diagnosed based on the presence of one or more of its manifestations. Patients with rosacea may have different triggering factors for the manifestations. These triggering factors may include, for example, any of genetic disposition, gastrointestinal disturbances (including dyspepsia with gastric hypochlorhydia and infestation with microaerophilic gram-negative bacteria *Helicobacter pylori),* hypertension, *Demodex folliculorum* mites, psychogenic factors, spicy foods, blushing, flushing, ultraviolet radiation, wind exposure, and stress. Often patients with rosacea are particularly susceptible to blushing and flushing, and signs of this may be an indicator of the probability of rosacea suffering later in life.

There are typically four stages of rosacea, as well as a predisposition to the condition. The stages can be defined as follows:

Pre-rosacea: skin flushes easily and redness lasts longer than normal and there is a family history of the condition.
Stage I: Frequent flushing, some persistent erythema.
Stage II: Persistent erythema and telangiectasias.
Stage III: Papules and pustules (plus Stage II).
Stage IV: Rhinophyma (bumpy, bulbous nose).

While certain lesions of rosacea may mimic lesions of acne vulgaris, the processes are separate and distinct. The principal differences between the two skin conditions are the presence of comedones (whiteheads and blackheads) in acne vulgaris only and not in rosacea, the characteristic mid-facial localization and flushing of rosacea not seen in acne, and the potential for eyelid involvement in rosacea which never occurs in acne. In fact, the clinical observation has been made that people who have classic acne vulgaris as teenagers rarely, if ever, develop full-blown rosacea as adults.

In the classic situation, rosacea is most common in adults between the ages of 20 and 84. For example, 63% of those people suffering from rosacea are between the ages of 20 and 59, while 27% of those people suffering from rosacea are between the ages of 60 and 84.

A further age breakdown shows that 19.1% of people suffering from rosacea are between the ages of 20 and 39; 47.4% are between the ages of 40 and 59; and 27.4% are between the ages of 60 and 84. Accordingly, the majority of rosacea sufferers have an age of at least 40 years.

The underlying cause of rosacea is presently unknown and has been a frequently-discussed medical topic, with little consensus having ever been reached. However, at least four factors or co-factors have been suggested.

The first of these factors is endocrine related, as rosacea tends to occur most frequently in women. As such, one definite type of rosacea is believed to have a hormonal basis.

A second suggested factor is vasomotor lability, believed to have some connection with menopause, which brings about an impairment of normal or consistent flow of blood to the face and its capillaries. Excessive flow of blood to the face, i.e., the well-known "hot flashes" of menopause, is believed to constitute a factor in the disease and its pathogenesis. More particularly, it has been proven that increased skin temperature, as occurs in facial flushing, increases susceptibility to the condition.

The prominent presence of erythema (redness) and flushing of the face of affected persons with aggravation from heat, sunshine (particularly due to UV light), cold, chemical irritation, emotions, spices, coffee, tea, and alcohol, particularly in persons with a fair complexion, has focused attention on this vasomotor aspect of the disease. However, treatment with medications to block such vasomotor flushing has often had no effect on other aspects of the disease such as papules and pustules. Further, rosacea-afflicted skin is abnormally sensitive to chemical and physical insults, while the frequent flushing and blushing in rosacea eventually leads to permanent skin redness.

As a third suggested factor, rosacea has been observed as a side effect or immune response to the use of certain cortisone products or standard acne medications, which can bring about a severe form of the condition. When topically applied to rosacea-affected skin, these medications generally irritate the skin and induce rosacea flare-ups. Similarly, agents that dilate blood vessels when ingested, for instance, ethanol and certain medications for high blood pressure, can bring on a rosacea blush when ingested by a person affected with rosacea. However, if untreated, rosacea can result in swollen veins, scattered lumps, and clusters of pustules on the face.

Finally, pathology analysis of the expressed contents of inflamed pustule follicles of the nose in acute rosacea has demonstrated the existence of demodices, which is a signature of the ectoparasite *Demodex folliculorum.* Accordingly, in such cases, a specific external pathogenic factor is evident. This factor is not present in acne.

Dietary avoidance of spicy foods and alcohol which cause flushing have in the past provided at most temporary symptomatic relief from rosacea. Jansen and Plewig, "Rosacea", *Clinical Dermatology* (Philadelphia: Lippincott-Raven Publishers, 1997; chapter 10-7) provide an excellent review of various treatments for rosacea in this regard.

Several potential treatments for rosacea have been disclosed in the art. However, none of these treatments have proven to be particularly effective. For example, U.S. Patent No. 5,654,013 discloses a method of reducing inflammation in rosacea involving lightly rubbing a block of crystalline sodium chloride over moistened skin in affected areas. No claim was made for any antibiotic effect on bacteria or ecto-parasites in the skin.

U.S. Patent No. 3,867,522 similarly discloses the abrasive use of sodium chloride crystals rubbed over affected skin in acne and related disorders, again with no intended antibiotic effect and with the goal of treatment being the lessening of the severity of the disease and not a permanent or even a temporary cure.

Rosacea has also previously been treated with oral and/or topical antibacterial agents. The oral antibiotics used include tetracycline, erythromycin, and minocycline. This antibiotic treatment has been shown to effectively block progression of rosacea through a poorly-understood anti-inflammatory mechanism, but studies have shown that these medications do not act by killing either bacteria or *Demodex folliculorum* organisms in affected skin.

One particular antibiotic disclosed in U.S. Patent No. 5,952,372 as effective for the oral treatment of rosacea is ivermectin (22,23-dihydroavermectin B1). However, it is uncertain whether ivermectin is orally effective in killing De*modex folliculorum,* as the patent alleges.

Azoles, e.g. metronidazole and imidazoles, have also been previously used as treatments for rosacea, particularly for moderate to severe rosacea.

In some instances, patents with rosacea have been successfully treated with retinoids (such as 0.025% tretinoin cream). There is preliminary evidence that 0.2% isotretinoin in a bland cream, which is less irritating than tretinoin, suppresses inflammatory lesions in stages II and III. Other patients have found relief with oral retinoids (e.g. isotretinoin capsules, such as Accutane® (Roche Laboratories, Nutley, NJ)).

Various topical antibiotic compositions used for the potential treatment of acne are known in the art. Some of these topical compositions have separately contained the antibiotics tetracycline, erythromycin, and clindamycin, as well as benzoyl peroxide, which exerts its antibacterial action via its potent oxidizing properties. However, these topical compositions were previously unknown as effective in treating rosacea. Further, the strong oxidizing properties of the peroxide component can result in unstable compositions. Benzoyl peroxide also can act as a sebosuppressant, an irritant, and a comedolytic agent.

These topical compositions combining at least two active antibacterial agents typically have the further drawback of requiring compounding by the pharmacist and must be refrigerated. After three months of room temperature storage, the compositions lose potency and effectiveness and must be replaced with a new batch.

For example, a currently available combination product is Benzamycin® brand topical gel (Dermik Laboratories, Berwyn, PA) which contains 3% of erythromycin and 5% of benzoyl peroxide. Benzamycin®, however, has several drawbacks. First, the product is supplied to pharmacies as a benzoyl peroxide gel in a first container and erythromycin powder in a second container. The product thus requires compounding by the pharmacist, who must (1) dissolve the erythromycin in alcohol, (2) add the erythromycin solution to the gel, and (3) stir until homogeneous in appearance. Second, the alcohol present in the composition as dispensed amounts to 16% of the total composition, which has proven to be excessively drying and irritating to the skin, particularly in combination with the benzoyl peroxide. Third, the composition as dispensed by the pharmacist (i.e., after reconstitution or compounding) lacks the stability necessary for extended storage at room temperature. The combination product can be stored under refrigeration for up to three (3) months.

Similarly, the currently available combination product BenzaClin® (Dermik Laboratories, Berwyn, PA) is a topical gel containing 1% of clindamycin and 5% of benzoyl peroxide. BenzaClin®, however, also has several drawbacks. For example, the product must be compounded by a pharmacist since it is supplied to pharmacies as a benzoyl peroxide gel in a first container and clindamycin powder in a second container. Accordingly, it lacks the stability necessary for extended storage at room temperature since the combined product can only be stored for up to two (2) months. By requiring compounding by pharmacists, it also has variability/impurity problems, which are the result of the drug forming partially dissolved or undissolved aggregates. This causes some patients to report that the product sometimes feels "gritty" when applied to the skin, further exacerbating the inflammation and irritation problem due to skin abrasion. Lastly, this composition must be topically applied at least twice a day to be effective in accordance with label directions.

Another currently available product, Cleocin T® brand clindamycin phosphate topical solution by Pharmacia & Upjohn Company of Kalamazoo, MI, is a topical solution containing 1% of clindamycin phosphate. Cleocin T®, however, has several drawbacks. For one, the formulation contains 50% isopropyl alcohol and water. This formulation often proves to be excessively drying and irritating to the skin. Second, the composition as dispensed by the pharmacist lacks the stability necessary for extended storage at room temperature.

For these reasons, there remains a need for storage-stable topical compositions that are effective in treating rosacea. In particular, it would be desirable to provide products combining the activity of an antibiotic compound, such as clindamycin, with the activity of benzoyl peroxide, for the treatment of rosacea, with few or none of the disadvantages described above. Such compositions should overcome the formulation and stability problems which have been associated with the prior compositions, and provide improved compositions which are less irritating, easy to formulate, have a smooth consistency after formulation, are substantially uniform, are adequately stable, and have a sufficiently long storage life with or without refrigeration.

### SUMMARY OF THE INVENTION

The present subject matter relates to a method for treating rosacea in a patient, comprising:
topically administering to a patient in need thereof a topical composition in an amount effective to treat said rosacea, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea.

In a preferred embodiment, the present subject matter also relates to a method for reducing or eliminating mite organisms that cause rosacea in a patient, comprising:
topically administering to skin of a patient affected with said mite organisms a topical composition in an amount effective to reduce or eliminate said mite organisms, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in reducing or eliminating said mite organisms.

In another preferred embodiment, the present subject matter relates to a method for treating rosacea in a patient having sensitive skin, comprising:
topically administering to sensitive skin areas, irritated skin areas, or inflamed skin areas of a patient in need thereof a topical composition in an amount effective to treat said rosacea, wherein said composition comprises:
   a storage-stable mixture of a benzoyl peroxide dispersion, clindamycin or a pharmaceutically acceptable salt or ester thereof, and a pharmaceutically acceptable carrier;
wherein said topical composition has a viscosity that enhances the effectiveness of the topical composition in treating rosacea.

In yet another preferred embodiment, the present subject matter relates to a method for treating rosacea in a patient, comprising:
topically administering to a patient in need thereof a topical composition in an amount effective to treat said rosacea, wherein said composition comprises:
   a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea;
and wherein said topical composition is administered concomitantly or sequentially with an additional active agent effective to treat said rosacea.

In still yet another preferred embodiment, the present subject matter relates to the use of benzoyl peroxide and clindamycin or a pharmaceutically acceptable salt or ester thereof in the preparation of a medicament in the form of a topical composition for treating rosacea in a patient, wherein said topical composition comprises:
an amount effective to treat said rosacea of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea.

In a further preferred embodiment, the present subject matter relates to the use of benzoyl peroxide and clindamycin or a pharmaceutically acceptable salt or ester thereof in the preparation of a medicament in the form of a topical composition for reducing or eliminating mite organisms that cause rosacea in a patient, wherein said topical composition comprises:
an amount effective to reduce or eliminate said mite organisms of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in reducing or eliminating said mite organisms.

In another preferred embodiment, the present subject matter relates to the of benzoyl peroxide and clindamycin or a pharmaceutically acceptable salt or ester thereof in the preparation of a medicament in the form of a topical composition for treating rosacea in a patient, wherein said topical composition comprises:
an amount effective to treat said rosacea of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea and wherein said composition is formulated to be suitable for topical application concomitantly or sequentially with an additional active agent effective to treat said rosacea.

In yet another preferred embodiment, the present subject matter relates to the use of benzoyl peroxide and clindamycin or a pharmaceutically acceptable salt or ester thereof in the preparation of a medicament in the form of a topical composition for treating rosacea in a patient having sensitive skin, wherein said topical composition comprises:
an amount effective to treat said rosacea of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea and wherein said composition is formulated to be suitable for topical application to sensitive skin areas, irritated skin areas, or inflamed skin areas of said patient.

### DETAILED DESCRIPTION OF THE INVENTION

### Definitions

The term "commercial purposes" refers to any purposes requiring any length of time or storage condition in accordance with the U.S. Food and Drug Administration (FDA) rules or regulations, including shipping time, storage, distribution, and refrigeration.

The term "rosacea" as used herein refers to a chronic recurring skin syndrome of unknown pathogenesis that generally but not exclusively involves the midline regions of the face encompassing varied combinations of clinical stigmata that include vascular instability (transient erythema [flushing]), vascular ectasia (non-transient erythema, telangiectasia), inflammatory lesions (papules, pustules and nodules), edema, skin thickening, ocular and rhinophyma changes. The full scope of rosacea, including its causes, symptoms, and effects, has previously been discussed by Wilkin J.K., "Rosacea. Pathophysiology and treatment", *Arch. Dermatol.,* 1994, 130, pp. 359-62; Wilkin J. et al., "Standard classification of rosacea: Report of the National Rosacea Society Expert Committee on the Classification and Staging of Rosacea", *J. Am. Acad. Dermatol.,* 2002, 46, pp. 584-7; and Dahl M.V. et al., "Temperature regulates bacterial protein production: Possible role in rosacea", *J. Am. Acad. Dermatol.,* 2004, 50, pp. 266-72, the contents for each of which are incorporated herein by reference in their entirety.

The term "rosacea NOS" as used herein refers to non-specified rosacea.

The term "acne rosacea rhinophyma" as used herein refers to a thickening of the nasal skin tissue with noticeable follicular pore (patulous) accentuation, surface nodularity, and abnormal enlargement that may occur in some patients as a part of the rosacea syndrome.

The term "perioral dermatitis" as used herein refers to an eruption involving the skin around the mouth and chin consisting of tiny microvesicles, scaling and peeling that does not have the vascular instability and other primary characteristics of rosacea. The full scope of perioral dermatitis, including its causes, symptoms, and effects, has previously been discussed by Hogan D.J. et al., "Perioral dermatitis: an uncommon condition?", CMAJ, 1986, 134, pp. 1025-8; and Laude T.A. et al., "Perioral dermatitis in children", *Semin. Cutan. Med. Surg.,* 1999, 18, pp. 206-9, the contents for each of which are incorporated herein by reference in their entirety.

The term "rhinophyma" as used herein refers to a thickening of the nasal skin tissue with more noticeable follicular pore (patulous) accentuation, surface nodularity and abnormal enlargement that may occur in due to tumors, in some patients as a part of the rosacea syndrome or infiltrative skin conditions.

The term "sensitivity" refers to the degree of skin irritation or skin inflammation, as exemplified by parameters in suitable assays for measuring sensitivity, inflammation, irritation, and the like. One such assay is the Jordan-King assay, as set forth in Jordan, W.P. 1994, Jordan/King modification of the Draize Repeat Insult Patch Test, Clairol Study #94046, Test Dates 10/3/94-11/11/94, the entire contents of which are hereby incorporated by reference.

### Compositions

The present subject matter relates to methods of using various topical compositions for treating rosacea in a patient. These topical compositions preferably comprise a storage-stable mixture of a benzoyl peroxide dispersion, clindamycin or a pharmaceutically acceptable salt or ester thereof, and a pharmaceutically acceptable carrier. Further, the topical composition preferably has a viscosity that enhances the effectiveness of the topical composition in treating rosacea.

The benzoyl peroxide component of the compositions used herein is introduced as a dispersion. The benzoyl peroxide included in this dispersion is pharmaceutical grade benzoyl peroxide. Further, the benzoyl peroxide in the dispersion may be in the form of a slurry of a finely divided powder, or in the form of a hydrous granular material. Preparation of suitable benzoyl peroxide constituents is well described in the medical and patent literature.

The benzoyl peroxide component of the compositions used herein is generally present in an amount of between about 0.5% to about 10% by weight of the total composition of benzoyl peroxide. In a preferred embodiment, the compositions used herein contain from about 1% to about 6.25% by weight of the total composition of benzoyl peroxide. In a particularly preferred embodiment, the compositions used herein contain about 1.5% to about 3.5% by weight of benzoyl peroxide. The compositions used herein are unobvious in that they can be produced having a standard deviation of benzoyl peroxide present within ± 0.07.

Additionally, the compositions used herein effectively maintain a benzoyl peroxide composition having not more than 0.01% by weight of benzoyl peroxide impurities, excluding solvents.

In a preferred embodiment, the benzoyl peroxide used is about 65% to about 80% pure, the remainder being purified water.

Prior to mixing, the benzoyl peroxide dispersion has a preferred viscosity of about 60,000 to about 250,000 centipoises. In a particularly preferred embodiment, the benzoyl peroxide dispersion has a viscosity of about 110,000 to about 220,000 centipoises prior to mixing.

The clindamycin component of the compositions used in the present methods is preferably a pharmaceutical grade salt or ester of clindamycin. Pharmaceutically acceptable salts, esters, or solvates of clindamycin refer to those which possess the desired pharmacological activity and which are neither biologically nor otherwise undesirable. The salts, esters, or solvates can be formed with inorganic or organic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride, hydrobromide, hydroiodide, 2-hydroxyethanesulfonate, lactate, maleate, methanesulfonate, naphthylate, 2-naphthalenesulfonate, nicotinate, oxalate, sulfate, thiocyanate, tosylate and undecanoate.

Base salts, esters, or solvates useful herein include ammonium salts, alkali metal salts such as lithium, sodium and potassium salts, alkaline earth metal salts such as calcium and magnesium salts, salt with organic bases such as dicyclohexylamine salts, N-methyl-D-glucamine, and salts with amino acids such as arginine, lysine, and so forth. Also the basic nitrogen-containing groups can be quarternized with such agents as: 1) lower alkyl halides, such as methyl, ethyl, propyl, and butyl chloride, bromides and iodides; 2) dialkyl sulfates like dimethyl, diethyl, dibutyl and diamyl sulfates; 3) long chain alkyls such as decyl, lauryl, myristyl and stearyl substituted with one or more halide such as chloride, bromide and iodide; and 4) aryl or arylalkyl halides like benzyl and phenethyl bromide and others.

Clindamycin phosphate (ester) and clindamycin hydrochloride (salt) are preferred pharmaceutically acceptable salts and esters of clindamycin which can be used in the present compositions due to their compatibility with gelling agents and extensive history of topical use.

The clindamycin component of the compositions used herein is generally present at an amount of from about 0.90% to about 2.5% by weight of the total composition. In a preferred embodiment, the compositions used herein contain between about 0.5% and about 1.5% by weight clindamycin. In a particularly preferred embodiment, the compositions used herein contain about 1.2% by weight clindamycin. The compositions used in the present methods are unique in that they can be produced having a standard deviation of clindamycin present within ± 0.015.

Additionally, the compositions used herein effectively maintain a clindamycin composition having not more than 0.02% by weight of clindamycin degradates.

In the final composition, the ratio of benzoyl peroxide to clindamycin may be from about 1.8:1 to 12:1. Particularly useful are compositions wherein the ratio of benzoyl peroxide to clindamycin is from about 4:1 to about 5:1. Further, the final compositions preferably have a final viscosity of about 50,000 to about 200,000 centipoises. In a particularly preferred embodiment, the final compositions have a final viscosity of about 100,000 to about 180,000 centipoises.

In a particularly preferred embodiment of the compositions used herein, the final topical composition has a viscosity lower than the viscosity of the benzoyl peroxide dispersion before mixing. This final viscosity that is lower than the viscosity of the benzoyl peroxide dispersion demonstrates that the compositions used in the present methods are easier to mix together, contain less degradates, and have a greater degree of uniformity than those compositions previously known in the art.

In another preferred embodiment, the final compositions exhibit a final pH of about 4.5 to about 5. In a particularly preferred embodiment, the final compositions exhibit a final pH of about 4.6 to about 4.8. This narrowly tailored pH is in part responsible for the advanced storage stability of the present compositions in comparison to those previously known in the art. In view of these particular viscosity and pH features, the compositions used in the present methods are storage-stable for commercial purposes.

In a preferred embodiment, the compositions used in the present methods are particularly stable when stored at a temperature of less than about 30 °C.

The present compositions do not require compounding at the time of dispensing and maintain stability indefinitely depending on the storage temperature, despite the relative incompatibility of benzoyl peroxide and clindamycin. This represents a distinct advantage over the formulations previously known in the art.

The present methods preferably use compositions formulated for either once-per-day or twice-per-day administration. In a preferred embodiment, the once-per-day administration is in the morning or A.M. to increase patient compliance and to account for skin conditions most favorable to reducing inflammation. An additional advantage of administration in the morning is the minimization of the risk of bleaching fabrics occasionally seen when a patient puts a benzoyl peroxide product on their face at night and the medication comes in contact with a "colored" pillow case or sheet, etc. resulting in a white spot on the fabric.

The benzoyl peroxide dispersion, as well as the final composition, may take the form of a gel, cream, lotion, suspension, emulsion, ointment, foam, or mixtures thereof. Other cosmetic treatment compositions known to those skilled in the art, including liquids and balms, are additionally contemplated as falling within the scope of the present subject matter. Further, the present subject matter contemplates applying any of these compositions with an applicator. Non-limiting examples of useful applicators include a pledget, a pad, and combinations thereof. Additionally, the present subject matter further contemplates that any of these topical composition are provided in a package of less than 5 g topical composition as a unit of use.

Emulsions, such as oil-in-water or water-in-oil systems, as well as a base (vehicle or carrier) for the topical formulation is selected to provide effectiveness of the active ingredient and/or avoid allergic and irritating reactions (e.g., contact dermatitis) caused by ingredients of the base or by the active ingredients.

Creams useful in the compositions used herein may also be semisolid emulsions of oil and water. They are easily applied and vanish when rubbed into the skin.

Lotions useful in the compositions used herein include suspensions of powdered material in a water or alcohol base (e.g., calamine), as well as water-based emulsions (e.g., some corticosteroids). Convenient to apply, lotions are also cool and help to dry acute inflammatory and exudative lesions.

Suitable lotions or creams containing the active compound may be suspended or dissolved in, for example, a mixture of one or more of the following: mineral oil, sorbitan monostearate, polysorbate 60 (polyoxyethylene 20 sorbitan monostearate), cetyl ester wax, cetearyl alcohol, 2-octyldodecanol, benzyl alcohol, and water.

Ointments which are useful herein are oleaginous and contain little if any water; feel greasy but are generally well tolerated; and are best used to lubricate, especially if applied over hydrated skin. These ointments are preferred for lesions with thick crusts, lichenification, or heaped-up scales and may be less irritating than cream formulations for some eroded or open lesions (e.g., stasis ulcers). Drugs in ointments are often more potent than in creams.

The compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water.

In severe cases, occlusive therapy may be useful herein, particularly where the trunk or extremities are affected by the rosacea. Covering the treated area with a nonporous occlusive dressing increases the absorption and effectiveness of topical corticosteroids. Usually, a polyethylene film (plastic household wrap) is applied overnight over cream or ointment, since a cream or ointment is usually less irritating than lotion in occlusive therapy. Plastic tapes may be impregnated with drug and is especially convenient for treating isolated or recalcitrant lesions; children and (less often) adults may experience pituitary and adrenal suppression after prolonged occlusive therapy over large areas.

Suitable gelling agents which may be useful in the present compositions include aqueous gelling agents, such as neutral, anionic, and cationic polymers, and mixtures thereof. Exemplary polymers which may be useful in the instant compositions include carboxy vinyl polymers, such as carboxypolymethylene. A preferred gelling agent is Carbopol® brand polymer such as is available from Noveon Inc., Cleveland, OH. Suitable gelling agents include Carbopol® polymers. Carbopol® polymers are high molecular weight, crosslinked, acrylic acid-based polymers. Carbopol® homopolymers are polymers of acrylic acid crosslinked with allyl sucrose or allylpentaerythritol. Carbopol® copolymers are polymers of acrylic acid, modified by long chain (C10-C30) alkyl acrylates, and crosslinked with allylpentaerythritol.

Other suitable gelling agents include cellulosic polymers, such as gum arabic, gum tragacanth, locust bean gum, guar gum, xanthan gum, cellulose gum, methylcellulose, hydroxyethylcellulose, hydroxypropylcellulose, and hydroxypropylmethylcellulose.

The compositions used herein may further contain at least one additional inactive ingredient in an amount effective to enhance the stability of said compositions. Any nontoxic, inert, and effective carrier may be used to formulate the compositions used herein. Well-known carriers used to formulate other therapeutic compounds for administration to humans will be useful in these compositions. Pharmaceutically acceptable carriers, excipients and diluents in this regard are well known to those of skill in the art, such as those described in The Merck Index, Thirteenth Edition, Budavari et al., Eds., Merck & Co., Inc., Rahway, N.J. (2001); the CTFA (Cosmetic, Toiletry, and Fragrance Association) In ternational Cosmetic Ingredient Dictionary and Handbook, Tenth Edition (2004); and the "Inactive Ingredient Guide", U.S. Food and Drug Administration (FDA) Center for Drug Evaluation and Research (CDER) Office of Management, January 1996, the contents of which are hereby incorporated by reference in their entirety. Examples of such useful pharmaceutically acceptable excipients, carriers and diluents include distilled water, physiological saline, Ringer's solution, dextrose solution, Hank's solution, and DMSO, which are among those preferred for use herein.

These additional components, as well as effective formulations and administration procedures, are well known in the art and are described in standard textbooks, such as Goodman and Gillman's: The Pharmacological Bases of Therapeutics, 8th Ed., Gilman et al. Eds. Pergamon Press (1990); Remington's Pharmaceutical Sciences, 17th Ed., Mack Publishing Co., Easton, Pa. (1990); and Harry's Cosmeticology, 8th ed. (2000, Chemical Publishing Co., Inc., New York, NY 10016), each of which are incorporated by reference herein in their entirety ingredients that can be used in the present compositions include but are not limited to carbomer, disodium monolauryl sulfosuccinate, disodium ethylenediaminetetraacetic acid (disodium EDTA), methyl paraben, poloxamer, glycerin, dimethicone, hydrated silica, sodium hydroxide, purified water, and mixtures thereof.

Other ingredients which may optionally be provided in the instant topical compositions include humectants, such as propylene glycol; solvents, such as alcohol (both organic and inorganic alcohol); and anti-microbial preservatives, such as methylparaben and propylparaben. The topical compositions may also include an organic or inorganic base, such as sodium hydroxide, which is used to adjust the pH of the initial components and the final product.

### Additional Active Ingredients

The subject matter described herein further contemplates administering an additional active ingredient readily known to those of skill in the art as useful in the topical treatment of skin disorders or conditions. These additional active ingredients are administered topically or orally either concomitantly or sequentially with the above described topical compositions comprising benzoyl peroxide and clindamycin for the treatment of rosacea. Accordingly, the additional active ingredient is administered with the topical composition either in adjunctive or co-therapy. That is, the additional active ingredient can either be administered as a component of the topical composition or as part of a second, separate composition. This second, separate composition can be either an oral or a topical composition.

Exemplary additional active ingredients include, but are not limited to, other macrolide antibiotics, bactericidal drugs, bacteriostatic drugs, cleansing agents, absorbents, anti-infective agents, anti-inflammatory agents, astringents (drying agents that precipitate protein and shrink and contract the skin), emollients (skin softeners), moisturizers, keratolytics (agents that soften, loosen, and facilitate exfoliation of the squamous cells of the epidermis), retinoids, salts thereof, and mixtures thereof.

Exemplary macrolide antibiotics contemplated herein include, but are not limited to, azithromycin, clarithromycin, erythromycin, lincomycin, doxycycline, minocycline, salts thereof, and mixtures thereof. The macrolides are similar in structure and activity. All the macrolides are easily absorbed and all are primarily bacteriostatic by inhibiting bacterial protein synthesis. These drugs are active against aerobic and anaerobic gram-positive cocci, with the exception of enterococci, and against gram-negative anaerobes, and can be useful herein.

Exemplary bactericidal drugs (i.e., they kill bacteria) contemplated herein include, but are not limited to, penicillins, cephalosporins, vancomycin, aminoglycosides, quinolones, polymyxins, salts thereof, and mixtures thereof.

Exemplary bacteriostatic drugs (i.e., they slow bacterial growth) contemplated herein include, but are not limited to, erythromycin, tetracyclines, chloramphenicol, lincomycin, clarithromycin, azithromycin, sulfonamides, salts thereof, and mixtures thereof. However, it is well know that some bactericidal drugs may be bacteriostatic against certain microorganisms and vice versa. These drugs are well known in the art and may be found, for example, in The Merck Manual of Diagnosis and Therapy, 13^{th} edition, Section 13, Chapter 153 Anti-bacterial Drugs, 2001, incorporated herein by reference in its entirety.

Other topical drugs known as useful for the treatment of rosacea are further contemplated herein for use in combination therapy with the present antimicrobial topical compositions. Non-limiting examples of such other topical drugs include sulfur, sodium sulfacetamide, retinoid compositions (such as, without limitation, natural retinoids, synthetic retinoids, retinoic acid, retinal, retinol, adapalene, tzarotene, isotretoin, their derivatives, isomers and analogs), azoles (such as, without limitation imidazoles, metronidazole), anti-inflammatory agents which are also antimicrobial agents ("anti-inflammatory and antimicrobial agents"), immunosuppressants (e.g. Pimecrolimus 1%), other agents which treat inflammation, rosacea lesions or manifestations, and/or possible infection associated with rosacea, or combinations thereof.

These additional active ingredients may be applied topically in the primary composition described herein, or in a separate composition, which is also topically applied. In another embodiment, the additional active ingredient may be provided to the rosacea sufferer in an oral composition. These topical and oral compositions may be administered simultaneously or in sequence.

Furthermore, the present formulations may be used with adjunct therapies and treatments, such as pre-washing with common soaps, and mild detergents. However, selection is important when treating skin disorders such as rosacea since antibacterial soaps and abrasive soaps may increase irritation and make it difficult to use follicular drugs. Such follicular drugs may include topical antibiotics and antiseptics, as well as intralesional corticosteroids.

In superficial pustular rosacea, the topical benzoyl peroxide/clindamycin compositions may be used in combination with one of the follicular drugs.

Another combination therapy involves 20% azelaic acid in cream form, which has antiproliferative and antibacterial effects.

An additional combination therapy contemplated herein is topical tretinoin (retinoic acid) in 0.025%, 0.05%, or 0.1% cream, 0.05% liquid, or 0.01% or 0.025% gel. Also, a new topical retinoid, Differin® brand adapalene 0.1% gel, Galderma Laboratories, San Antonio, TX, was recently approved in the USA and may be useful since it may be slightly less irritating than topical tretinoin. Other retinoids which may be useful in combination therapy include Panretin®, containing alitretinoin, and Targretin®, containing bexarotene, Ligand Pharmaceuticals Inc., San Diego, CA. Since retinoids must be applied carefully and at night to avoid excessive irritation, a regimen in combination with these drugs may be used over time to achieve results. For example, retinoid therapy may be initiated and then followed on with once a day treatment in accordance with the present methods. Exposure to sunlight when using retinoids and concurrent use of other drugs are restricted to prevent severe irritation. However, a back-to-back alternating regimen over a period of weeks or months time may be useful. With tretinoin or adapalene, rosacea may worsen at first; improvement usually requires at least 3 to 4 weeks of treatment.

Other topical drugs including OTC drugs, various sulfur-resorcinol combinations, and oral antibiotics may also be helpful in combination with the present compositions when treating rosacea.

Accordingly, a preferred embodiment of the present subject matter additionally relates to a method for the treatment of rosacea in a patient in need thereof, comprising administering a combination of benzoyl peroxide and clindamycin to said patient, wherein said combination contains a low level of lincomycin phosphate sulfoxide and lincomycin sulfoxide, i.e. components that can be derived from clindamycin, or can be modified to form clindamycin.

### Methods of Treating Rosacea

The compositions described herein are preferably topically administered to skin of a patient affected by rosacea. In preferred aspects, the patient is a human.

Over one million human patients (actually almost 1.5 million human patients) are known to suffer from rosacea in its various forms. Based on data available for 2002, 75.4% of the total rosacea patients suffer from rosacea NOS, 12.5% of the total rosacea patients suffer from acne rosacea rhinophyma, 11.6% of the total rosacea patients suffer from perioral dermatitis, and 0.4% of the total rosacea patients suffer from rhinophyma.

This population of rosacea patients can further be broken down by gender. For example, based on the same 2002 data, 65.0% of the rosacea NOS patients are female, 31.3% are male, and 3.7% are of an unspecified gender; 68.2% of the acne rosacea rhinophyma patients are female and 31.8% are male; 70.5% of the perioral dermatitis patients are female, 22.8% are male, and 6.8% are of an unspecified gender; and 50.9% of the rhinophyma patients are female and 49.1% are male. Based on this data, then, rosacea tends to occur in females at least twice as much as in males. Accordingly, particularly preferred embodiments of the present subject matter contemplate methods of treating rosacea in a patient, wherein the patient is a human female.

Likewise, this data for rosacea patients for 2002 can further be broken down by age. For example, based on the 2002 data, 47.4% of the rosacea NOS patients are 50-59 years of age, 19.1% are 20-39 years of age, 13.3% are 65-74 years of age, 7.8% are 60-64 years of age, 6.3% are 75-84 years of age, 5.7% are of an unspecified age, and 0.4% are 85 years of age or older; 58.4% of the acne rosacea rhinophyma patients are 40-59 years of age, 22.5% are 20-39 years of age, 7.4% are 65-74 years of age, 5.4% are of an unspecified age, 4.2% are 75-84 years of age, and 2.0% are 85 years of age or older; 51.3% of the perioral dermatitis patients are 20-39 years of age, 20.2% are 40-59 years of age, 11.5% are 10-19 years of age, 4.8% are 65-74 years of age, 4.1% are of an unspecified age, 2.9% are of 60-64 years of age, 2.8% are 75-84 years of age, and 2.4% are 3-9 years of age; and 50.9% of the rhinophyma patients are 65-74 years of age and 49.1% are 20-39 years of age.

Based on this data, rosacea is shown to occur most frequently in patients having an age of between 20 and 84 years old. In fact, approximately 66% of rosacea cases occur in patients aged 20-59, while approximately 26% of rosacea cases occur in patents aged 60-84. Further, only about 19% of rosacea cases occur in patients having an age under 40 years old. Accordingly, particularly preferred embodiments of the present subject matter contemplate methods of treating rosacea in a patient, wherein the patient is a human of between 20 and 84 years old. In especially preferred embodiments, the patient will be a human of at least 40 years old or older.

Lastly, the data for rosacea patients for 2002 can be further broken down by specialty. In this regard, 77.4% of the rosacea NOS patients are diagnosed by dermatology, 8.8% are diagnosed by family practice, 7.9% are diagnosed by internal medicine, 2.3% are diagnosed by osteopathic medicine, 1.2% are diagnosed by ophthalmology, 0.4% are diagnosed by obstetrics/gynecology, 0.4% are diagnosed by geriatrics, 0.3% are diagnosed by allergy, and 0.3% are diagnosed by gastroenterology; 63.3% of the acne rosacea rhinophyma patients are diagnosed by dermatology, 13.5% are diagnosed by family practice, 11.3% are diagnosed by internal medicine, 6.2% are diagnosed by osteopathic medicine, 2.9% are diagnosed by obstetrics/gynecology, 1.8% are diagnosed by gastroenterology, 0.7% are diagnosed by allergy, and 0.5% are diagnosed by general practice; 81.6% of the perioral dermatitis patients are diagnosed by dermatology, 5.8% are diagnosed by family practice, 4.1% are diagnosed by internal medicine, 3.0% are diagnosed by general practice, 2.8% are diagnosed by osteopathic medicine, 1.9% are diagnosed by all other surgery, and 0.6% are diagnosed by allergy; and 50.9% of the rhinophyma patients are diagnosed by dermatology and 49.1% are diagnosed by family practice.

While not being limited to any specific cause for the rosacea, in a preferred embodiment the present methods involve the treatment of rosacea in each of these classes of patients that exhibits effects selected from the group consisting of mite organism infestation, erythema, prominent vascularization, dryness, papules, pustules, swelling, telangiectasia, hypertrophy of the sebaceous glands, nodules, flushing, blushing, rhinophyma, and combinations thereof.

In a particularly preferred embodiment, the mite organisms are *Demodex folliculorum.* By effectively reducing or eliminating the population of *Demodex* mites in affected skin areas, the present methods achieve a more complete remission of clinical signs and symptoms of the disease than any previously described method.

*Demodex folliculorum* is an ectoparasite in the mite family. Accordingly, the treatment herein is capable of eradicating the entire life cycle of such a microscopic insect, including egg, larval, and adult stages. For this reason, several doses of the present compositions are preferably applied to the patient over an extended period of time to allow time for *Demodex* eggs to hatch into immature mites that are killed before they can mature into egg-producing adults. In preferred embodiments, the present compositions are administered for at least two weeks on a regular basis to alleviate and/or eliminate the rosacea. For severe case of rosacea, the present compositions are preferably applied on a regular basis for at least 5 weeks to eliminate the rosacea.

After elimination of the rosacea, application of the present topical compositions may continue once a day to maintain the skin as rosacea-free.

After the present compositions carry out their miticidal activity on skin *Demodex folliculorum* organisms, inflammatory responses to the organisms begin to diminish. However, remnants of the dead mites still elicit some flushing and lesion formation until the cleanup processes of the body fully remove them, a process that can at times require six to eight weeks. After prolonged intervals of freedom from rosacea symptoms, should classic signs begin to reappear, treatment can be repeated. Such retreatments should not be necessary more than one or two times per year.

In a further preferred embodiment, the present methods involve treating rosacea in a patient having sensitive skin. In this regard, the present topical compositions are topically applied to sensitive skin areas, irritated skin areas, or inflamed skin areas.

In another preferred embodiment, the topical application of the present compositions reduces the redness, flushing, and blushing associated with either rosacea or sensitive skin.

The treatment for rosacea described herein can also be effective in treating other skin disorders or conditions associated with, or commonly further occurring in skin affected by, rosacea. These other skin disorders or conditions can include but are not limited to microbial infections and inflammation of tissue. The microbial infections can be caused by gram-positive bacteria, gram-negative bacteria, and mixtures thereof. Exemplary specific bacteria include but are not limited to P. *acnes, Strep. Pyogenes, Staph. Aureus, E. coli, Pseudonomas originosa,* and mixtures thereof.

Exemplary specific other skin disorders associated with, or commonly further occurring in skin affected by, rosacea treatable herein include but are not limited to acne, impetigo, atopic dermatitis, secondary skin infections, seborrhea, skin lesions, and bacterial skin infections. In a preferred embodiment, these other skin disorders or conditions improve following treatment with the present compositions.

In another preferred embodiment, the present subject matter further relates to a method for the treatment of rosacea in a patient in need thereof, comprising administering a combination of benzoyl peroxide and clindamycin which has been refrigerated to said patient. This combination has a specific degradation profile, in accordance with the data submitted below.

### Process for Preparing

The present subject matter further relates to a process for preparing a storage-stable topical composition for treating rosacea, which comprises the steps of:
forming at a temperature of about 15 to about 25 °C a benzoyl peroxide intermediate dispersion having between about 5.9% and about 7.2% benzoyl peroxide and having a viscosity of about 60,000 to about 250,000 centipoises;
forming at a temperature of about 15 to about 25 °C a clindamycin intermediate solution sufficient to yield a composition which contains between about 0.5% and about 1.5% by weight clindamycin active in the final product; and
mixing said benzoyl peroxide intermediate dispersion and said clindamycin intermediate solution under conditions sufficient to yield a benzoyl peroxide and clindamycin mixture having a final pH of between about 4.5 to about 5.0,
wherein said mixture has a viscosity of about 50,000 to about 200,000 centipoises, and wherein said composition comprises sufficient inactive ingredients to provide storage stability and effectiveness for a treatment period.

The mixture made according to this process preferably comprises a benzoyl peroxide gel intermediate mixed with a clindamycin solution intermediate. The benzoyl peroxide gel intermediate preferably contains between about 5.9% and about 7.2% by weight benzoyl peroxide.

Also, the composition is preferably manufactured to have about 1% to about 3% less water by weight as compared to a topical formulation having one of benzoyl peroxide or clindamycin alone, but not both together. Such formulations unexpectedly result in compositions that exhibit less skin sensitivity.

Referring to the formulation process of the present compositions, a gel is initially formed. The gel is composed of a carbomer, disodium monolauryl sulfosuccinate, and disodium EDTA to which methylparaben is added as a preservative. Purified water is used as a diluent.

After the gel is formed, wetting agents and emollients are added. After the pH is adjusted, the active ingredients are added to form the final compound.

As discussed above, the active ingredients can be added to the inert ingredients at the same time or separately.

The resultant combination maintains stability for a minimum of three months at room temperature (e.g. 22°C) and relative, or ambient, humidity.

### Routes of Administration/Dosage

To be effective, the route of administration for the compositions used in the present methods must readily affect the target areas. In particular, rosacea is known to affect the face, eyelids, nose, trunk, and extremities.

Dosage levels for the antibiotics and the benzoyl peroxide are well known in the art and are selected to maximize the treatment of the above conditions. The specific dose level for any particular patient will vary depending upon a variety of factors, including the activity of the specific compound employed; the age, body weight, general health, sex and diet of the patient; the time of administration; the rate of excretion; drug combination; the severity of the particular disease being treated; and the form of administration. Typically, in vitro dosage-effect results can provide useful guidance on the proper doses for patient administration. Studies in animal models are also helpful. The considerations for determining the proper dose levels are well known in the art and are incorporated herein for the present subject matter.

Pharmacokinetic parameters such as bioavailability, absorption rate constant, apparent volume of distribution, unbound fraction, total clearance, fraction excreted unchanged, first-pass metabolism, elimination rate constant, half-life, and mean residence time are well known in the art.

Lessening exposure by once-daily administration affects multiple pharmacokinetic parameters and provides the initial mechanism for avoiding skin irritation and inflammation and the other toxicity issues discussed herein. Additional formulations may be prepared which factor in the benefit/risk ratio for a clindamycin and benzoyl peroxide composition. The level of toxicity of these compounds is known and reference is made to the package inserts for Cleocin T® and Benza-Clin® and the level of adverse events reported from their clinical trials. In particular, BenzaClin® reported having the following events: dry skin (12%), pruritis (2%), peeling (2%), erythema (1%) and sunburn (1%) as compared to vehicle which reported dry skin (6%), pruritis (<1%), peeling (-), erythema (<1%) and sunburn (-), or roughly twice the number of side effects as vehicle.

Since benzoyl peroxide is a keratolytic, i.e. causes softening and swelling of the cells at the surface of the skin so that the outer layer of the skin peels off or can easily be removed, reducing exposure to it reduces irritation. Upon application, the benzoyl peroxide converts to benzoic acid and has anti-bacterial and anti-fungal properties. Additionally, the low pH of the present formulations may have an additive keratolytic effect on the skin as well as on the anti-bacterial properties. Benzoyl peroxide may also acts as a preservative within the formulation. Clindamycin may degrade at pH higher than pH 6, thus requiring the pH to be maintained below this level, as described herein. The present formulations take these and other factors into account and are manufactured to reduce sensitivity, irritation, and/or inflammation.

Single dosage kits and packages containing once per day amount of composition may be prepared. Single dose, unit dose, and once-daily disposable containers of the present compositions are contemplated as within the scope of the present subject matter.

The present compositions may be formulated for storage in a substantially non-reactive laminated package to enhance stability of the package. This new method of storage provides enhanced package stability in comparison with the previous paper-based packages.

The amount of composition per single packet may range be from about 0.1 mL to about 20.0 mL, preferably between about 0.5 and about 5.0 mL, more preferably between about 1 and about 3 mL.

In particular, the ability to formulate compositions capable of long term storage, without pre-mixing or compounding requirements prior to application, are also contemplated. Specifically, the present compositions remain unexpectedly stable in storage for periods including between about 3 and about 18 months, preferably between about 3 and about 15 months, more preferably between about 3 and about 12 months, and alternately any time period between about 6 and about 18 months. In this regard, while the product may be refrigerated during the distribution and pharmacy storage phases, the product does not need refrigeration for the about 3 months and longer as stated above when stored by the patient at room temperature.

Once-daily disposable packaging may also improve patient compliance, especially for teenagers.

The stability and effectiveness of the topical preparations may last for at least 3 to 18 months at ambient or room temperature. It has been found that the greater the amount of clindamycin in the final product, the greater the stability is maintained. Stability is maintained indefinitely under refrigeration because degradation is slowed through the storage temperature. This improved stability provides pharmacists and other dispensers of medication with a product which no longer requires compounding at the time of dispensing. Because compounding is no longer required, homogeneity is controlled at the point of manufacture, which improves dosing and ultimately compliance. Furthermore, the present compositions do not employ alcohol as a diluent, which eliminates the drying or irritating effects commonly associated therewith.

Stability of the composition is maintained for longer periods of time depending on the amount of clindamycin employed in the final product and the ratio of benzoyl peroxide to clindamycin. For example, when 1.2% of clindamycin is present in the composition, the shelf life can reach from seven to fourteen months at room temperature while maintaining effectiveness. In contrast, when only 1.02% of clindamycin is employed, the shelf life of the product is closer to three months.

Differences in packaging components and manufacturing techniques yield varied formula responses over a period ranging between seven and fourteen months in stability testing as evidenced by the following data:

| Ref No. | BPO/Clindamycin Ratio | Minimum Projected Stability |
|---|---|---|
| A | 5/1.2 | 14 months |
| B | 5/1.2 | 9 months |
| C | 5/1.2 | 7 months |
| D | 5.9/1 | 7 months |
| E | 5/1.02 | 3 months |
| F | 5/1.02 | 3 months |
| BPO = benzoyl peroxide | | |

In addition to the amount of clindamycin as a control over degradation, the temperature at which the composition is stored determines the length of time that the composition remains stable. When the composition is stored at a temperature below ambient temperature (25° C), the stability is maintained indefinitely. For example, storing the compound at 6° C with the proper amount of excess of clindamycin results in an anticipated shelf life of 3 to 5 years.

Advantageously, the final product requires no compounding by the pharmacist. In addition, compliance with exact amounts is possible with a lessened chance of impurities entering the product and contaminating it.

By maintaining the compositions at the present specific pH, the tendency of benzoyl peroxide to oxidize and degrade clindamycin is largely overcome and the product remains stable during storage at room temperature for extended periods, typically several months or longer. Additionally, the present compositions have been found to remain substantially odor free even after storage at room temperature for extended periods. This is surprising since clindamycin solutions frequently develop a strong offensive odor upon aging. The presence of such an odor is unacceptable in topical formulations which are to be applied to a patient's face.

The following examples are illustrative of preferred embodiments and are not to be construed as limiting the subject matter expressed herein. All polymer molecular weights are mean average molecular weights. All percentages are based on the percent by weight of the final delivery system or formulation prepared unless otherwise indicated and all totals equal 100% by weight.

In the examples, the following ingredients are used: carbomers and polymeric emulsifiers (polyacrylates), such as for example Carbopol® 940 from Noveon Inc., Cleveland, OH; disodium monolauryl sulfosuccinate, such as Monamate™ LA-100, Uniqema, New Castle, DE; emulsifier-solubilizer-stabilizers block PEG/PPG co-polymers such as poloxamer 182, also known as Pluracare® L-62, BASF Corporation, Parsippany, NJ; surfactant-emollient-lubricant-plasticizers such as dimethicone also known as Dow Fluid 200®, Dow Corning Corporation, Midland, MI; sequestering agents such as disodium EDTA; and hydrated silica and absorbants.

### EXAMPLE 1

A highly stable gel composition is prepared using the following components. The active ingredients are benzoyl peroxide and clindamycin phosphate. The formulation is prepared to contain 5% by weight benzoyl peroxide and 1.2% by weight clindamycin phosphate as a gel. The remaining components are inert or auxiliary.

| Intermediate Gel Preparation | |
|---|---|
| Ingredient | Parts by Weight |
| Gel: | |
| Purified Water | 86.50% |
| Carbomer | 2.00% |
| Disodium monolauryl sulfosuccinate | 0.04% |
| Disodium EDTA | 0.10% |
| Methylparaben | 0.30% |
| Total: | 88.94% |

The gel is combined with the following to produce the instant composition:

| Formulation to be Administered | |
|---|---|
| Wetting Agents and Emollients: | |
| Poloxamer 182 | 0.20% |
| Glycerin | 4.00% |
| Dimethicone | 0.10% |
| Hydrated Silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | 5.00% |
| Clindamycin Phosphate | 1.20% |
| Total: | 6.20% |
| | |
| Total for Composition: | 100.00% |

### EXAMPLE 2

The following composition is obtained when the following component formulations are mixed in equal parts, and later combined to yield the highly stable product.

| Intermediate Gel Preparation | |
|---|---|
| Ingredient | Parts by Weight |
| Gel: | |
| Purified Water | 82.70% |
| Carbomer | 2.00% |
| Disodium monolauryl sulfosuccinate | 0.04% |
| Disodium EDTA | 0.10% |
| Methylparaben | 0.12% |
| Total: | 85.14% |

The gel is combined with the following to produce the instant composition:

| Benzoyl Peroxide Formulation | |
|---|---|
| Wetting Agents and Emollients: | |
| Poloxamer 182 | 0.20% |
| Glycerin | 4.00% |
| Dimethicone | 0.10% |
| Hydrated silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | 10.00% |
| Clindamycin Phosphate | --- |
| Total: | 10.00% |
| | |
| Total for Composition: | 100.00% |

| Clindamycin Formulation | |
|---|---|
| Ingredient | Parts by Weight |
| Gel: | |
| Purified Water | 90.30% |
| Carbomer | 2.00% |
| Disodium monolauryl sulfosuccinate | 0.04% |
| Disodium EDTA | 0.10% |
| Methylparaben | 0.30% |
| Total: | 92.74% |

The gel is combined with the following to produce the instant composition:

| Formulation to be Administered | |
|---|---|
| Wetting Agents and Emollients: | |
| Poloxamer 182 | 0.20% |
| Glycerin | 4.00% |
| Dimethicone | 0.10% |
| Hydrated silica | 0.25% |
| Total: | 4.55% |
| | |

| pH Adjustment: | |
|---|---|
| Sodium Hydroxide | 0.31% |
| Total: | 0.31% |
| | |

| Active Ingredients: | |
|---|---|
| Benzoyl Peroxide | --- |
| Clindamycin Phosphate | 2.40% |
| Total: | 2.40% |
| | |
| Total for Composition: | 100.00% |

The resultant mixture is essentially 10% of benzoyl peroxide with essentially 2% clindamycin.

### EXAMPLE 3

Tables 1 and 2 show the stability of the active ingredients. A fourteen-month analysis was performed on a 5.9% benzoyl peroxide and 1% clindamycin gel formulation. Measurements were taken at the end of 3 months and every month thereafter until the 8th month. No measurements were taken at 8 months. Thereafter, measurements were taken at 9, 12, and 14 months. The composition was stored at 3 different temperatures, i.e., 6° C, 25° C, and 30° C. The level of clindamycin was measured at each temperature, as well as the amount of benzoyl peroxide. The results are as follows:

**TABLE 1 -**

| **Clindamyoin stability** Benzoyl Peroxide 5% (5.9% in formula) and clindamycin 1% (1% in formula) | | | |
|---|---|---|---|
| Clindamycin Concentration (as % w/w) | | | |
| | 6° C | 25° C | 30° C |
| Initial | | 1.01 | |
| 3 months | 0.95 | 0.90 | 0.77 |
| 4 months | 1.01 | 0.95 | 0.79 |
| 5 months | 1.04 | 0.95 | 0.79 |
| 6 months | 0.96 | 0.91 | 0.71 |
| 7 months | 1.05 | 0.92 | 0.70 |
| 9 months | 1.03 | ND | ND |
| 12 months | 0.98 | 0.79 | 0.37 |
| 14 months | 0.98 | 0.76 | 0.27 |
| ND = No Data | | | |

**TABLE 2 -**

| Benzoyl Peroxide Stability Benzoyl Peroxide (5.9% in formula) and clindamycin 1% (1% in formula) | | | |
|---|---|---|---|
| Benzoyl Peroxide (BPO) Concentration (as % w/w) | | | |
| | 6° C | 25° C | 30° C |
| Initial | | 6.13 | |
| 3 months | 5.97 | 5.90 | 5.98 |
| 4 months | 6.07 | 6.05 | 5.98 |
| 5 months | 6.08 | 5.96 | 5.84 |
| 6 months | 6.13 | 6.04 | 5.91 |
| 7 months | 6.23 | 6.19 | 6.06 |
| 9 months | 6.02 | 5.95 | |
| 12 months | 5.95 | 5.89 | 5.63 |
| 14 months | 6.10 | 6.10 | 5.77 |

### EXAMPLE 4

Tables 3 and 4 show the stability of the active ingredients in the composition containing 5% of benzoyl peroxide and 1.2% of clindamycin.

A six-month analysis of the composition was undertaken following the procedure of Example 3 and utilizing a different amount of clindamycin and benzoyl peroxide.

**TABLE 3 -**

| **Clindamycin Stability** Benzoyl Peroxide 5% (BPO) (5.9% in formula) and clindamycin 1% (1.2% in formula) | | | |
|---|---|---|---|
| Clindamycin Concentration (as % w/w) | | | |
| | 6° C | 25° C | 30° C |
| Initial | | 1.24 | |
| 1 months | 1.25 | 1.24 | 1.15 |
| 2 months | 1.28 | 1.21 | 1.01 |
| 3 months | 1.23 | 1.13 | 0.94 |
| 6 months | 1.21 | 1.05 | ND |

**TABLE 4 -**

| Benzoyl Peroxide Stability Benzoyl Peroxide 5% (BPO) (5.9% in formula) and clindamycin 1% (1.2% in formula) | | | |
|---|---|---|---|
| Benzoyl Peroxide (BPO) Concentration (as % w/w) | | | |
| | 6° C | 25° C | 30° C |
| Initial | | 5.09 | |
| 1 months | 5.10 | 5.02 | 5.08 |
| 2 months | 5.25 | 5.20 | 5.13 |
| 3 months | 5.16 | 5.18 | 4.82 |
| 6 months | 5.07 | 5.06 | ND |
| ND = No Data | | | |

### EXAMPLE 5

Tables 5 through 13 show the stability of the active ingredients. An analysis of at least 24 months was performed following the procedure of Example 3. Measurements were taken after storage for a specified number of months at 6° C followed by storage for 91 days thereafter at 25° C.

The columns in the following tables represent the following components (as % w/w):
Column A - Clindamycin
Column B - Clindamycin HCl
Column C - Clindamycin B-2 Phosphate
Column D - Clindamycin Phosphate Sulfoxide Isomer 1
(Clindamycin Degradate 1)
Column E - Clindamycin Phosphate Sulfoxide Isomer 2
(Clindamycin Degradate 2)
Column F - Lincomycin Phosphate Sulfoxide (Clindamycin Degradate 3)

**TABLE 5 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 1 | | | | | | | | |
| | A | B | C | D | E | F | pH range | Package Size |
| Initial | 1.01 | <0.005 | 0.012 | 0.037 | 0.047 | ND | 4.6-4.7 | 5 g |
| 25 months | 0.95 | <0.005 | 0.008 | 0.041 | 0.074 | 0.00 7 | 4.6-4.7 | 5 g |
| 30 months | 0.93 | <0.005 | 0.007 | 0.044 | 0.082 | 0.00 3 | 4.6-4.7 | 5 g |
| 36 months | 0.93 | <0.005 | <0.001 | 0.050 | 0.086 | 0.00 3 | 4.6-4.7 | 5 g |
| 48 months | 0.89 | <0.005 | <0.001 | 0.062 | 0.111 | 0.00 3 | 4.6-4.8 | 5 g |

**TABLE 6 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 2 | | | | | | | | |
| | A | B | C | D | E | F | pH Range | Package Size |
| Initial | 1.02 | <0.005 | 0.013 | 0.011 | 0.016 | ND | 4.6-4.7 | 45 g |
| 21 months | 0.96 | <0.005 | <0.001 | 0.046 | 0.070 | 0.008 | 4.6-4.8 | 45 g |
| 24 months | 0.95 | <0.005 | 0.008 | 0.048 | 0.084 | 0.008 | 4.6-4.7 | 45 g |
| 30 months | 0.93 | <0.005 | 0.007 | 0.045 | 0.083 | 0.003 | 4.6-4.7 | 45 g |
| 36 months | 0.93 | <0.005 | <0.001 | 0.048 | 0.086 | 0.003 | 4.6-4.8 | 45 g |

**TABLE 7 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 3 | | | | | | | | |
| | A | B | C | D | E | F | pH Range | Package Size |
| Initial | 1.05 | <0.005 | 0.008 | <0.001 | 0.005 | ND | 4.8 | 45 g |
| 24 months | 0.92 | <0.005 | <0.001 | 0.044 | 0.079 | 0.00 3 | 4.6-4.8 | 45 g |
| 30 months | 0.93 | <0.005 | <0.001 | 0.048 | 0.087 | 0.00 2 | 4.7-4.8 | 45 g |
| 37 months | 0.91 | <0.005 | <0.001 | 0.055 | 0.095 | 0.00 3 | 4.5-4.9 | 45 g |
| 42 months | 0.89 | <0.005 | <0.001 | 0.058 | 0.106 | 0.002 | 4.7-4.8 | 45 g |
| 48 months | 0.89 | <0.005 | <0.001 | 0.058 | 0.11 0 | 0.003 | 4.6-4.7 | 45 g |

**TABLE 8 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 4 | | | | | | | | |
| | A | B | C | D | E | F | pH Range | Package Size |
| 24 months | 0.92 | <0.00 5 | <0.00 1 | 0.04 4 | 0.08 0 | 0.00 4 | 4.7-4.8 | 45 g |
| 30 months | 0.91 | <0.00 5 | <0.00 1 | 0.04 4 | 0.07 5 | 0.00 2 | 4.7-4.8 | 45 g |
| 37 months | 0.90 | <0.00 5 | <0.00 1 | 0.04 9 | 0.09 0 | 0.00 2 | 4.7-4.8 | 45 g |
| 42 months | 0.89 | <0.00 5 | <0.00 1 | 0.05 8 | 0.10 3 | 0.00 2 | 4.7-4.8 | 45 g |

**TABLE 9 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 5 | | | | | | | | |
| | A | B | C | D | E | F | pH Range | Package Size |
| Initial | 1.01 | <0.005 | 0.008 | <0.001 | 0.005 | ND | 4.7-4.9 | 5 g |
| 24 months | 0.91 | <0.005 | <0.001 | 0.044 | 0.078 | 0.004 | 4.6-4.8 | 5 g |
| 30 months | 0.90 | <0.005 | <0.001 | 0.040 | 0.073 | 0.002 | 4.7-4.8 | 5 g |
| 37 months | 0.88 | <0.005 | <0.001 | 0.052 | 0.093 | 0.002 | 4.4-4.8 | 5 g |
| 42 months | 0.89 | <0.005 | <0.001 | 0.057 | 0.104 | 0.002 | 4.7-4.8 | 5 g |

**TABLE 10 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 6 | | | | | | | | |
| | A | B | C | D | E | F | pH Range | Package Size |
| 24 months | 0.91 | <0.005 | <0.001 | 0.046 | 0.075 | 0.00 1 | 4.7-4.8 | 45 g |
| 31 months | 0.90 | <0.005 | <0.001 | 0.042 | 0.080 | 0.00 1 | 4.7-4.8 | 45 g |
| 36 months | 0.88 | <0.005 | <0.001 | 0.055 | 0.101 | 0.00 1 | 4.7-4.8 | 45 g |

**TABLE 11 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 7 | | | | | | | | |
| | A | B | C | D | E | F | pH Range | Package Size |
| Initial | 1.01 | <0.005 | <0.001 | 0.005 | 0.008 | 0.005 | 4.7 | 45 g |
| 24 months | 0.91 | <0.005 | <0.001 | 0.045 | 0.079 | <0.001 | 4.7-4.8 | 45 g |

**TABLE 12 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 8 | | | | | | | | |
| | A | B | C | D | E | F | pH Range | Package Size |
| Initial | 1.03 | <0.005 | <0.001 | 0.006 | 0.010 | 0.002 | 4.7 | 45 g |

**TABLE 13 -**

| **Benzoyl Peroxide/Clindamycin Stability** Benzoyl Peroxide 5% (BPO) and clindamycin 1% | | | | | | | | |
|---|---|---|---|---|---|---|---|---|
| Trial 9 | | | | | | | | |
| | A | B | C | D | E | F | pH Range | Package Size |
| Initial | 1.04 | <0.005 | <0.001 | 0.006 | 0.009 | 0.002 | 4.7-4.8 | 45 g |

The present subject matter being thus described, it will be obvious that the same may be modified or varied in many ways. Such modifications and variations are not to be regarded as a departure from the spirit and scope of the present subject matter and all such modifications and variations are intended to be included within the scope of the following claims.

## Claims

1. Use of benzoyl peroxide and clindamycin or a pharmaceutically acceptable salt or ester thereof in the preparation of a medicament in the form of a topical composition for treating rosacea in a patient, wherein said topical composition comprises:
an amount effective to treat said rosacea of a storage-stable mixture of a benzoyl peroxide dispersion, clindamycin or a pharmaceutically acceptable salt or ester thereof, and a pharmaceutically acceptable carrier,
wherein said topical composition has a viscosity that enhances the effectiveness of the topical composition in treating rosacea.

2. The use of claim 1, wherein said topical composition is formulated to be suitable for topical application concomitantly or sequentially with an additional active agent effective to treat said rosacea, preferably either in adjunctive or co-therapy.

3. The use of claim 2, wherein said additional active agent is selected from the group consisting of other macrolide antibiotics, bactericidal drugs, bacteriostatic drugs, cleansing agents, absorbents, anti-infective agents, anti-inflammatory agents, astringents, emollients, moisturizers, keratolytics, retinoids, salts thereof, and mixtures thereof.

4. The use of any of claims 1-3, wherein said use reduces or eliminates mite organisms that cause rosacea in said patient.

5. The use of any of claims 1-4, wherein said topical composition is formulated to be suitable for topical application to sensitive skin areas, irritated skin areas, or inflamed skin areas of said patient.

6. The use any of claims 1-5, wherein said topical composition has a viscosity lower than the viscosity of the benzoyl peroxide dispersion before mixing.

7. The use of any of claims 1-6, wherein said topical composition, after addition of the benzoyl peroxide dispersion, has a final viscosity of about 50,000 to about 200,000, preferably about 100,000 to about 180,000, centipoises.

8. The use of any of claims 1-7, wherein said benzoyl peroxide dispersion, prior to making said topical composition, has a viscosity of about 60,000 to about 250,000, preferably about 110,000 to about 220,000, centipoises.

9. The use of any of claims 1-8, wherein said topical composition has a final pH of about 4.5 to about 5, preferably about 4.6 to about 4.8.

10. The use of any of claims 1-9, wherein said topical composition is formulated for once-per-day or twice-per-day administration.

11. The use of any of claims 1-10, wherein said topical composition is selected from the group consisting of a gel, cream, lotion, suspension, emulsion, ointment, foam, and mixtures thereof.

12. The use of claim 11, wherein said topical composition is suitable for application with an applicator.

13. The use of any of claims 1-12, wherein said topical composition is provided in a package of less than 5 g topical composition as a unit of use.

14. The use of any of claims 1-13, wherein said patient is a human female.

15. The use of any of claims 1-14, wherein said topical composition is stored at a temperature of less than about 30 °C.

16. The use of any of claims 1-15, wherein said benzoyl peroxide is about 65% to about 80% pure.

17. The use of any of claims 1-16, wherein said mixture comprises about 0.5% to about 10% by weight, preferably about 1% to about 6.25% by weight, more preferably about 1.5% to about 3.5% by weight, benzoyl peroxide.

18. The use of any of claims 1-17, wherein said composition further contains at least one additional inactive ingredient in an amount effective to enhance the stability of said composition.

19. The use of claim 18, wherein said at least one inactive ingredient is selected from carbomer, disodium monolauryl sulfosuccinate, disodium ethylenediaminetetraacetic acid (disodium EDTA), methyl paraben, poloxamer, glycerin, dimethicone, hydrated silica, sodium hydroxide, purified water, derivatives thereof, and mixtures thereof.

20. The use of any of claims 1-19, wherein said rosacea exhibits effects selected from mite organism infestation, De*modex folliculorum* infestation, erythema, prominent vascularization, dryness, papules, pustules, swelling, telangiectasia, hypertrophy of the sebaceous glands, nodules, flushing, blushing, rhinophyma, and combinations thereof.
